# EUROPEAN PATENT APPLICATION

(11) **EP 2 311 440 A1**
(43) Date of publication of application: **20.04.2011**
(21) Application number: 10190392.0
(22) Date of filing: 05.04.2002
(51) Int. Cl.: A61K 9/22, A61K 9/26, A61K 9/52, A61K 31/00

(54) **Controlled delivery of tetracycline compounds and tetracycline derivatives**

(30) Priority: 05.04.2001 US 281854 P
(62) Divisional of application: 02725534.8
(71) Applicant: Collagenex Pharmaceuticals, Inc., Newtown, PA 18940 (US)
(72) Inventor: Ashley, Robert A., Newton, PA 18940 (US)
(74) Representative: Jansen, Cornelis Marinus

(57) **Abstract**

A composition is provided for delivering a tetracycline compound to a mammal. The composition includes an antibiotic tetracycline compound and a controlled-release agent having at least one controlled-release agent. The tetracycline compound is associated with the controlled- release matrix to provide a release profile whereby the mammal is treated substantially without antibiotic activity. Methods for treating a mammal with a tetracycline compound and a dosage unit are also provided utilizing the controlled-release tetracycline composition.

## Description

### CROSS-REFERENCE TO RELATED APPLICATION

This application is a divisional of European Application No. 02725534.8 This application claims the benefit of U. S. Provisional Application No. 60/281,854, filed April 5,2001, which is incorporated herein by reference.

### BACKGROUND OF INVENTION

The invention relates to delivering a tetracycline compound to a mammal.

More specifically, the invention relates to controlled release of a tetracycline compound or derivative thereof for treatment of a mammal in the absence of antibiotic activity (i. e. antimicrobial activity).

Tetracycline and a number of its chemical relatives form a particularly successful class of antibiotics. Certain of the tetracycline compounds, including tetracycline itself, as well as sporocycline, etc., are broad spectrum antibiotics, having utility against a wide variety of bacteria.

Conventional tetracycline compositions are designed to optimize their antibiotic properties. The conventional compositions operate by creating a spike in serum concentration followed by a rapid diminution in serum concentration.

Accordingly, relatively high doses are administered which have a short serum concentration half-life. This short serum half-life requires the conventional compositions to be administered often, e. g every 3-6 hours.

Tetracyclines have been described as having a number of other therapeutic uses in addition to their antibiotic properties. For example, tetracyclines are also known to inhibit the activity of collagen destructive enzymes such as mammalian collagenase, gelatinase, macrophage elastase and bacterial collagenase. Golub et al., J. Periodont. Res. 20:12-23 (1985); Golub et al. Crit. Revs. Oral Biol. Med. 2: 297-322 (1991);U.S. Pat. Nos. 4,666,897; 4,704,383; 4,935,411; 4,935,412. In addition, tetracyclines have been known to inhibit wasting and protein degradation in mammalian skeletal muscle, U.S. Pat. No. 5,045,538.

Furthermore, tetracyclines have been shown to enhance bone protein synthesis in U.S. Pat. No. Re. 34,656, and to reduce bone resorption in organ culture in U.S. Pat. No. 4,704,383.

Similarly, U.S. Pat. No. 5,532,227 to Golub et al, discloses that tetracyclines can ameliorate the excessive glycosylation of proteins. In particular, tetracyclines inhibit the excessive collagen cross linking which results from excessive glycosylation of collagen in diabetes.

These properties cause the tetracyclines to be useful in treating a number of diseases. For example, there have been a number of suggestions that tetracyclines, including non-antibiotic tetracyclines, are effective in treating arthritis. See, for example, Greenwald et al., "Tetracyclines Suppress Metalloproteinase Activity in Adjuvant Arthritis and, in Combination with Flurbiprofen, Ameliorate Bone Damage," Journal of Rheumatology 19:927-938(1992); Greenwald et al., "Treatment of Destructive Arthritic Disorders with MMP Inhibitors: Potential Role of Tetracyclines in, Inhibition of Matrix Metalloproteinasas:Therapeutic Potential," Annals of the New York Academy of Sciences 732: 181-198 (1994); Kloppenburg et al., "Minocyaline in Active Rheumatoid Arthritis," Arthritis Rheum 37:629-636(1994); Ryan et al., "Potential of Tetracycline to Modify Cartilage Breakdown in Osteoarthritis," Current Opinion in Rheumatology 8: 238-247(1996); O'Dell et al., "Treatment of Early Rheumatoid Arthritis with Minocycline or Placebo," Arthritis Rheum 40:842-848(1997).

Tetracyclines have also been suggested for use in treating skin diseases. For example, White et al., Lancet, Apr. 29, p. 966 (1989) report that minocycline is effective in treating dystrophic epidermolysis bullosa, which is a life-threatening skin condition believed to be related to excess collagenase.

The effectiveness of tetracycline in skin disorders has also been studied by Elewski et al., Journal of the American Academy of Dermatology 8:807-812 (1983). Elewsld et al, disclosed that tetracycline antibiotics may have anti-inflammatory activity in skin diseases.

Similarly, Plewig et al., Journal of Investigative Dermatology 65:532 (1975), disclose experiments designed to test the hypothesis that antibiotics are effective in treating inflammatory dermatoses. The experiments of Plewig et al. establish that tetracyclines have anti-inflammatory properties in treating pustules induced by potassium iodide patches.

The use of tetracyclines in combination with non-steroidal anti-inflammatory agents has been studied in the treatment of inflammatory skin disorders caused by acne vulgaris. Wong et al., Journal of American Academy of Dermatology 1: 1076-1081 (1984), studied the combination of tetracycline and ibuprofen and found that tetracycline was an effective agent against acne vulgaris while ibuprofen was useful in reducing the resulting inflammation by inhibition of cycloxygenase. Funt et al., Journal of the American Academy of Dermatology 13: 524-525 (1985), disclosed similar results by combining antibiotic doses of minocycline with ibuprofen. similar results by combining antibiotic doses of minocycline with ibuprofen.

An antibiotic tetracycline derivative, doxycycline, has been used to inhibit nitrate production. D'Agostino et al., Journal of Infections Diseases: 177:489-92 (1998), disclose experiments where doxycycline, administered to mice injected with bacterial lipopolysaccharide (hereinafter LPS), exerted a protective effect by inhibiting nitrate production by an IL-10 independent mechanism.

Therefore, there are numerous uses for tetracycline compounds aside from their antibiotic activity. While tetracycline antibiotics are generally effective for treating infection, the use of these compounds can lead to undesirable side effects. For example, the long term administration of antibiotic tetracyclines can reduce or eliminate healthy biotic flora, such as intestinal flora, and can lead to the production of antibiotic resistant organisms or the overgrowth of yeast and fungi.

Accordingly, there is a need for a composition for improve delivery of tetracycline compounds to a mammal that, unlike conventional compositions, provides a dosage below that which is required for an antibiotic response in the mammal at a relatively constant serum level with a longer serum half-life.

### SUMMARY OF INVENTION

The present invention includes a composition for delivering a tetracycline compound to a mammal. The composition includes an antibiotic tetracycline compound and at least one controlled-release agent. The tetracycline compound is associated with the controlled-release agent to provide a tetracycline-release profile characterized by delivery of a dose below that which is required for antibiotic activity (i.e, antimicrobial activity) such that the mammal is treated with a tetracycline compound substantially without antibiotic activity.

The amount of the tetracycline compound released by the composition can vary, as long as it is below the threshold blood serum concentration level required for antibiotic activity. In general, the blood serum level will be between about 0.1 and 1.0 µg/ml, preferably between about 0.3 and 0.8 µg/ml. This release profile should be maintained at a substantially constant rate for between about 6-24 hours.

In a preferred embodiment, the tetracycline is doxycycline. The preferred blood serum level of doxycycline is 0.4-0.8 µg/ml. over a period of 12-24 hours.

The composition also can include a controlled-release agent selected from the group consisting of an instantaneous-release agent, a sustained-release agent, a delayed-release agent, and combinations thereof. In one embodiment, the composition can contain all three release agents associated with the tetracycline compound to provide a substantially constant dosage rate over a designated time period.

The present invention also includes a method of treating a mammal with a tetracycline compound. The method includes administering to the mammal a tetracycline compound which is associated with at least one controlled-release agent to provide a release profile having nonantibiotic activity over a pre-selected time period, preferably 6-24 hours.

The method also can include a controlled-release agent selected from the group consisting of an instantaneous-release agent, a sustained-release agent, a delayed-release agent, and combinations thereof. In one embodiment, the composition can contain all three release agents associated with the tetracycline compound to provide a substantially constant dosage rate over a designated time period.

A unit dosage is also provided for controlled delivery of a tetracycline compound. The unit dosage includes a tetracycline compound and at least one controlled-release agent. The tetracycline compound is associated with the controlled-release agent to provide a tetracycline release profile in the mammal substantially without antibiotic activity. In preferred embodiments, the unit dosage is either a capsule or a tablet.

The composition for delivering a tetracycline compound to a mammal and the corresponding method of treating a mammal with a tetracycline compound, as described herein, provides a number of benefits over conventionally utilized controlled delivery compositions for administration of a tetracycline compound.

First, by administering the tetracycline compound in a dose below that which is necessary to provide an antibiotic response, undesirable side effects, such as the reduction of healthy flora in the body, the production of antibiotic resistant organisms, or the overgrowth of opportunistic yeast and fungi, are avoided.

Second, the controlled release composition of the invention increases patient compliance. Instead of administering a low dose of a tetracycline compound many times during the day, the composition of the invention allows the patient to administer the tetracycline compound one or two times a day. The controlled release of the tetracycline compound creates the desired dose profile below that which is necessary for an antibiotic response in the mammal.

The composition of the invention also avoids the reduction in tetracycline uptake after eating. Very often, with conventional tetracycline compounds, the percentage of the tetracycline compounds reaching the bloodstream from the GI tract will decrease once the mammal begins eating. This reduction in tetracycline uptake is ameliorated with a composition that can be taken once or twice a day, especially with a controlled release formula that can remain entrapped in the upper portion of the GI tract as opposed to the small intestine.

Additionally, because the serum concentrations with the composition of the invention remain substantially lower than peak serum concentrations from an equivalent dosage administered as an immediate release formulation, the risk of phototoxicity encountered with conventional tetracycline compositions is reduced.

### BRIEF DESCRIPTION OF THE DRAWINGS

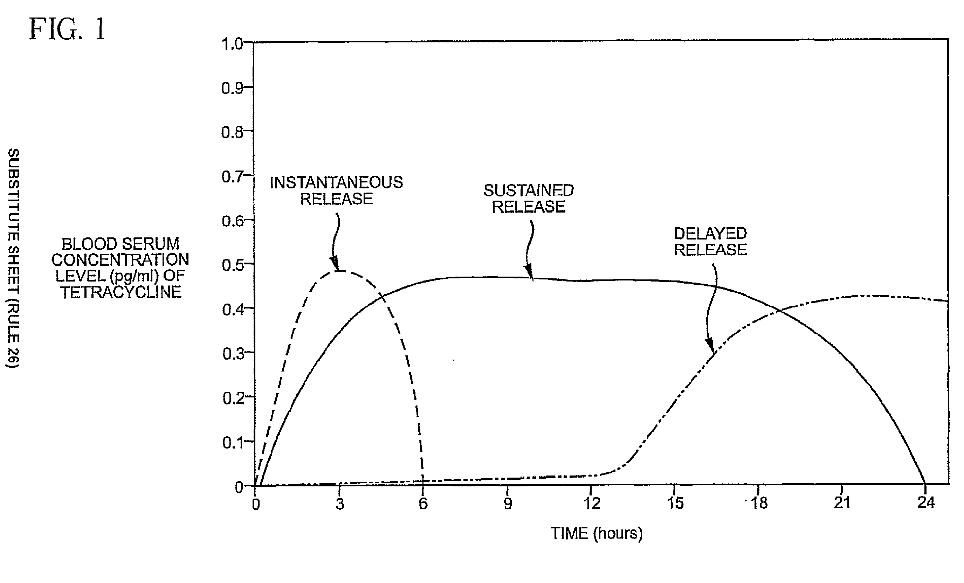

Figure 1 depicts a tetracycline release profile utilizing a combination of three different controlled-release agents which are associated with a tetracycline compound in a composition according to the present invention.

### DETAILED DESCRIPTION OF INVENTION

The composition of the invention is designed to provide a release profile that is the direct opposite of the conventional profile, described above. More specifically, the composition of the invention provides for the controlled release of a tetracycline compound to a mammal whereby there is substantially no antibiotic activity in the mammal. The composition of the invention provides its therapeutic effect by providing a dose of the tetracycline compound below that which is required to produce an antibiotic effect in the mammal at a substantially constant rate over a longer period of time, e.g. 12-24 hours.

The composition of the invention is administered to a mammal. Mammals include, for example, humans, as well as pet animals such as dogs and cats, laboratory animals such as rats and mice, and farm animals such as horses and cows.

"Tetracycline compound" as defined herein refers to tetracycline or any tetracycline derivative, as described above, possessing antibiotic activity when administered above the required serum level threshold, as is known in the art.

The parent compound, tetracycline, has the following general stricture:

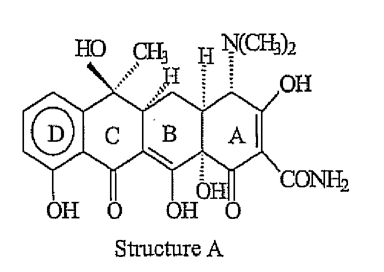

The numbering system of the multiple ring nucleus is as follows:

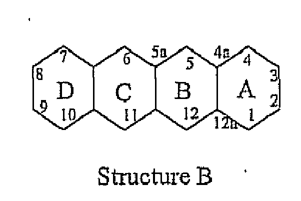

Tetracycline, as well as the 5-OH (oxytetracycline, e.g. Terramycin) and 7-Cl (chloxotatracycline, e.g. Aureomycin) derivatives, exist in nature, and are all well known antibiotics. Semisynthetic derivatives such as 7-dimethylamino-tetracycline (minocycline) and 6α-deoxy-5-hydroxy-tetracycline (doxycycline) are also known tetracycline antibiotics. Natural tetracyclines may be modified without losing their antibiotic properties, although certain elements of the structure must be retained to do so. Preferred antibiotic tetracyclines include tetracycline, doxycycline, demeclocycline, minocycline, and lymecycline.

A class of compounds has also been defined which are structurally related to the antibiotic tetracyclines, but which have had their antibiotic activity substantially or completely expunged by chemical modification. The modifications that may and may not be made to the basic tetracycline structure were reviewed by Mitscher, L.A., The Chemistry of the Tetracycline Antibiotics, Marcel Dekker, New York (1978), Ch. 6. According to Mitscher, the modification at positions 5-9 of the tetracycline ring system can be made without causing the complete loss of antibiotic properties. However, changes to the basic structure of the ring system, or replacement of substituents at positions 1-4 or 10-12, generally lead to synthetic tetracyclines with substantially less, or essentially no, antibacterial activity.

The composition of the invention can include, in addition to the tetracycline compound, one or more other therapeutic agents. The combination of the tetracycline compound with such other agents can potentiate the therapeutic protocol. The composition of the invention can also include a combination of the tetracycline compound in a suitable pharmaceutical carrier (vehicle) or excipient as understood by practitioners in the art.

In addition to the tetracycline compound, the composition of the invention includes at least one controlled-release agent. Controlled-release agents are known in the art. See for example, U.S. PatentNos. 4,837,030; 5,262,164; 5,582,837; 5,681,585; 5,716,631; 5,736,152; 5,840,332; 5,855,915; 6,007,843; 6,020,002; 6,120,803; and 6,143,353.

The composition of the invention can include various relative amounts of the tetracycline compound and the controlled release agent. For example; the tetracycline can make up 10%, 20%, 30%, 40%, 50%, 60%, 70%, 80% or 90% of the composition. The controlled release agent can make up 10%, 20%, 30%, 40%, 50%, 60%, 70%, 80% or 90% of the composition.

The tetracycline compound is associated with the controlled release matrix to provide a tetracycline-release-profile in the mammal whereby the mammal is treated with the tetracycline compound substantially without antibiotic activity. It is preferred that the controlled-release matrix be capable of releasing the tetracycline compound in an amount and at a rate sufficient to maintain an effective tetracycline blood serum level over a designated time period.

The tetracycline and controlled-release agent are associated with each other physically (e.g., by mechanical means such as mixing, mulling, compacting, etc.) and/or chemically, such as by chemical reaction, and/or secondary chemical bonding, e.g., Van der Waals forces, etc. The tetracycline compound/controlled-release agent combinations are included in the invention composition in an amount sufficient to provide a highly predictable pre-selected release profile of the therapeutically active tetracycline as a result of normal interaction of the mammal biosystem on the tetracycline/controlled-release matrix system combination.

The controlled-release agent can include one or more ingredients for controlling the rate at which the tetracycline component is made available to biological system of the mammal. The controlled-release agent can include an instantaneous release agent, a delayed release agent, a sustained release agent, or any combination thereof.

An instantaneous release agent refers to an ingredient which promotes or enhances immediate release to the mammal. The instantaneous release agent can be an additional ingredient that enhances dispersion of the tetracycline compound. An example of an instantaneous release agent is a surfactant.

A sustained release agent is an ingredient, or combination of ingredients, which permits release of the tetracycline compound to the mammal at a certain level over a period of time. Examples of sustained release agents include gels, waxes, fats, emulsifiers, combinations of fats and emulsifiers, polymers, starch, cellulose polymers, etc., as well as combinations thereof. The sustained release agent can also include, for example, the above in combination with other polymeric or biodegradable coatings or matrices.

A delayed release agent is an ingredient which prevents the tetracycline compound from being made available to the mammal until some time after initial administration. The delayed release agent prevents release of the tetracycline compound until some time in the future. Examples of delayed release agents include, but are not limited to, polymeric or biodegradable coatings or matrices, including cellulose polymers, and combinations thereof.

In a preferred embodiment, the composition of the invention comprises more than one controlled-release agent, and can include, all three types of controlled-release agents, i.e., an instantaneous release agent, a sustained release agent, and a delayed release agent. Using all three types ofcontrolled-release agents can produce a profile that administers the tetracycline compound in a specific dose over an extended period of time, e.g., 12-24 hours. Figure 1 depicts a release profile utilizing an instantaneous, delayed, and sustained controlled-release agent.

The sustained controlled-release agent preferably consists of a cellulose polymer, preferably a high molecular weight cellulose polymer, selected from the group consisting of hydroxypropyl methyl cellulose (HPMC), hydroxyethyl cellulose (HEC), hydroxypropyl cellulose (HPC), carboxy methyl cellulose (CMC), and mixtures thereof. Of these, the most preferred water soluble cellulose polymer is HPMC.

Preferably the HPMC is a high molecular weight HPMC, with the specific molecular weight selected to provide the desired release profile. For example, a tablet designed to provide a substantially constant release rate over a 12 hour period will preferably contain HPMC having an average molecular weight of at least about 65,000, more preferably about 85,000.

The controlled-release component can also contain minor amounts of other materials which can affect the release profile. Examples of such materials include conventional waxes and waxy materials used in pharmaceutical formulations, such as canuba wax, spermaceti wax, candellila wax, cocoa butter, cetosteryl alcohol, beeswax, partially hydrogenated vegetable oils, ceresin, paraffin, myristyl alcohol, stearyl alcohol, cetyl alcohol and stearic acid. Hydrophilic gums are also contemplated for use, in minor amounts, which can have an effect on the release profile. Examples of hydrophilic gums include acacia, gelatin, tragacanth, veegum, xanthin gum, carboxymethyl cellulose (CMC), hydroxy propyl cellulose (HPC) and hydroxy ethyl cellulose (HEC).

The tetracycline composition of the invention can be administered in the form of a liquid as a suspension or solution, or alternatively in solid form, such as a tablet, pellet, particle, capsule, or soft gel. For example, the form can be polymeric capsules filled with solid particles which can, in turn, be made to release the tetracycline compound according to a known pattern or profile. Such particles can also be made to have more than one release profile so that over an extended time the combined release patterns provide a pre-selected profile.

In one embodiment, the tetracycline compound/controlled-release agent combination is administered in the form of a heterogeneous matrix, such as, for example, a compressed tablet, to control the release of the tetracycline compound either by diffusion, erosion of the matrix or a combination of both.

Other combinations of controlled release agent and tetracycline compound contemplated by the invention include a combination of polymeric material(s) and tetracycline compound which is formed into a sandwich, and which relies on, at least the physical disintegration actions of diffusion or erosion to contralledly release the tetracycline. Additionally, heterogeneous dispersions or solutions of tetracycline in water-swellable hydrogel matrices are useful in controlling the release of the tetracycline by slow surface-to-center swelling of the matrix and subsequent release of the tetracycline by a combination of diffusion of the tetracycline from the water-swollen part of the matrix and erosion of the water-swollen matrix containing the tetracycline.

The sustained controlled-release agent will preferably provide for a sustained release of tetracycline according to a desired release profile through the use of one or more of the release ingredients described above. More preferably, the controlled-release agent will provide a release profile which releases the tetracycline compound at a substantially constant rate over a designated time period whereby the mammal is treated with the tetracycline substantially without antibiotic activity.

As the terminology is used herein, "substantially constant rate" refers to maintaining a release rate of the active ingredient, i.e., tetracycline, within a desired range over at least about 60 % of the designated time period for release, preferably over at least about 70 %, more preferably over at least about 80 % of the designated time period, and most preferably over about 90%.

The release profile in the composition of the invention provides substantially no antibiotic activity. In other words, the dosage of the tetracycline compound administered by the release profile is below the amount required for antibiotic activity.

For example, an antibiotic tetracycline compound of the invention is advantageously administered in an amount that results in a serum tetracycline concentration which is 10-80% of the minimum antibiotic serum concentration. The minimum antibiotic serum concentration is the lowest concentration known to exert a significant antibiotic effect.

Some examples of the plasma antibiotic threshold levels of tetracyclines based on steady-state pharmacokinetics are as follows: 1.0 µg/ml for doxycycline; 0.8 µg/ml for minocycline; and 0.5 µg/ml for tetracycline.

The amount administered will vary depending on various factors as is known in the art, such as the size of the mammal, the specific tetracycline compound used, etc. The amount can be determined by one skilled in the art.

In general, the amount of the tetracycline compound released will provide a blood serum level of tetracycline that has the desired therapeutic activity, but no antibiotic activity. Some examples of blood serum levels of tetracycline include a minimum of about 0.1µg/ml, preferably about 0.3 µg/ml; and a maximum of about1.0 µg/ml, more preferably about 0.8 µg/ml. For example, when the tetracycline compound utilized is doxycycline, it is preferred that a serum of about 0.4 to about 0.8 µg/ml be maintained.

The controlled release agent in the composition is designed to maintain the specified serum concentration levels over an extended period of time, for example 6, 8, 12, or 24 hours at a substantially constant rate. It is preferred that the controlled release agent release the tetracycline compound in the mammal to provide the specified sub-antibiotic serum concentration levels for at least 12-24 hours.

Other ingredients can be used in accordance with the present invention to improve the tetracycline composition. Such ingredients include binders, which contribute to the ease of formation and general quality of the tablet; lubricants, which aid in compressing and compacting the tablet; and flow agents or glidants, which adhere to the cohesive material in order to enhance flow properties by reducing interparticle friction.

Examples of useful binders include calcium sulfate, calcium carbonate, microcrystalline cellulose, starches, lactose, sucrose, mannitol, sorbitol, polyvinylpyrrolidone, methylcellulose, sodium carboxymethyleellulose, ethylcellulose, polyacrylamides, polyvinyloxaazolidone, and polyvinylalcohols. A preferred binder is microcrystalline cellulose, such as Avicel PH-101 sold by FMC Corporation.

Lubricants can include, but are not limited to, the following: magnesium stearate, calcium stearate, zinc stearate, stearic acid, hydrogenated vegetable oils, sterotex, polyoxyethylene, monostearate, talc, polyethyleneglycol, sodium benzoate, sodium lauryl sulfate, magnesium lauryl sulfate and light mineral oil. Of these, the preferred lubricants are magnesium stearate and stearic acid.

Flow agents or glidants which can be used include starch, talc, magnesium and calcium stearate, zinc stearate, dibasic calcium phosphate, magnesium carbonate, magnesium oxide, calcium silicate, silicon dioxide and silica aerogels. A preferred flow agent or glidant is silicon dioxide.

A tablet having sufficient mechanical strength and an acceptable release profile can be produced, for example, by mixing a powdered tetracycline compound with HPMC and suitable binders, lubricants and flow agents and compressing the mixture in a tablet press. A typical compression force used in forming the tablets is in the range of about 45 to about 56 KN, preferably about 50 to about 53 KN, to achieve a tablet having a hardness in the range of about 15 kp to about 30 kp, preferably about 18 kp to about 25 kp.

The invention is also directed to a unit dosage for controlled delivery of a tetracycline compound. The unit dosage utilizes the controlled-release tetracycline composition, as described above, to deliver the tetracycline compound to a mammal substantially without antibiotic activity in the mammal at a substantially constant rate over a designated time period. The unit dosage being administered can have a release time selected, for example, from about 6, 8, 12 and 24 hours. 12-24 hours is preferred.

The unit dosage provides a dosage of antibiotic tetracycline to create a blood serum tetracycline concentration of about 0.1 to about 1.0 µg/ml, more preferably about 0.3 to about 0.8 µg/ml. For example, when the tetracycline utilized is doxycycline, it is preferred that a serum of between about 0.4-0.8 µg/ml be maintained.

The unit dosage can be administered in the form of a liquid, for example, in a suspension or solution, or alternatively in solid form, such as a tablet, pellet, particle, capsule, or soft gel. A tablet or capsule is preferred.

One embodiment of the unit dosage is a capsule which contains beadlets. Within each capsule are beadlets which are coated with various coatings that dissolve at different pH levels.

A method is also provided herein for treating a mammal with tetracycline compounds. The method includes administering a tetracycline composition to a mammal as set forth above. The composition includes a tetracycline compound that can be an antibiotic tetracycline compound, non-antibiotic tetracycline compound, or combinations thereof. The composition also includes a controlled-release matrix having at least one controlled-release agent. The tetracycline compound is associated with the controlled-release matrix such that the mammal is treated with the tetracycline substantially without antibiotic activity.

Any suitable form of administration may be utilized. Systemic administration is preferred. Examples of systemic administration are enteral and parenteral.

Enteral administration is a preferred route of delivery of the tetracycline composition, and compositions including the tetracycline compound with appropriate diluents, carriers, and the like are readily formulated. Liquid or solid (e.g., tablets, gelatin capsules) formulations can be employed.

In a preferred embodiment, the controlled-release composition is entrapped in the upper portion of the gastrointestinal tract, for example, the stomach or duodenum. Such compositions are typically manufactured by utilizing controlled-release agents of a larger particle size, as is known in the art. It is preferred that at least 50%, more preferably greater than 80% of the tetracycline in the composition be released in the upper GI tract.

By entrapping the tetracycline composition in the upper portion of the GI tract, the loss of tetracycline uptake encountered after eating is diminished. Also, the loss of beneficial flora in the small and large intestine is reduced, as compared to conventional tetracycline compositions.

Parenteral use (e.g., intravenous, intramuscular, subcutaneous injection) is also contemplated, and formulations using conventional diluents, carriers, etc., such as are known in the art can be employed to deliver the compound.

In one embodiment of the invention, the tetracycline compound can be a non-antibiotic tetracycline compound or derivative. Non-antibiotic tetracycline compounds are structurally related to the antibiotic tetracyclines, but have had their antibiotic activity substantially or completely eliminated by chemical modification. For example, non-antibiotic tetracycline compounds are capable of achieving antibiotic activity comparable to that of tetracycline or doxycycline at concentrations at least about ten times, preferably at least about twenty five times, greater than that of tetracycline or doxycycline, respectively.

Examples of chemically modified non-antibiotic tetracyclines (CMTs) include 4-de(dimethylamino)tetracycline (CMT-1), tetracyclinonitrile (CMT-2), 6-demethyl-6-deoxy-4-de(dimethylamino)tetracycline (CMT-3), 7-chloro-4-de(dimethylamino)tetracycline (CMT-4), tetracycline pyrazole (CMT-5), 4-hydroxy-4-da(dimethylamino)tetracycline (CMT-6), 4-de(dimethylamino-12α-deoxytetracycline (CMT-7), 6-deoxy-5α-hydroxy-4-de(dimethylamino)tetracycline (CMT-8), 4-de(dimsthylainmo)-12α-deoxyanhydrotetracycline (CMT-9), 4-de(dimethylamino)minocycline (CMT-10).

Tetracycline derivatives, for purposes of the invention, maybe any tetracycline derivative, including those compounds disclosed generically or specifically in co-pending U.S. patent application serial no. 09/573,654 filed on May 18, 2000, which are herein incorporated by reference.

The invention is further described with reference to the following clauses.
1. A composition for delivering tetracycline compound to a mammal comprising:
   a. an antibiotic tetracycline compound, and
   b. at least one controlled-release agent;
   said tetracycline compound associated with said at least one controlled-release agent to provide a tetracycline-release-profile in said mammal, whereby said mammal is treated with said tetracycline compound substantially without antibiotic activity.
2. A composition as described in clause 1 wherein said release profile provides a blood serum concentration level of said tetracycline compound in said mammal of about 0.1 llg/ml to about 1.0 llg/ml.
3. A composition as described in clause 2 wherein said release profile provides a blood serum concentration level of said tetracycline compound in said mammal of about 0.3 llg/ml to about 0.8 llg/ml.
4. A composition as described in clause 1 wherein said release profile is maintained at a substantially constant rate for between about 6-24 hours.
5. A composition as described in clause 1 wherein said antibiotic tetracycline compound is selected from the group consisting of tetracycline, doxycycline, demeclocycline, minocycline, and lymecycline.
6. A composition as described in clause 5 wherein said tetracycline compound is doxycycline.
7. A composition as described in clause 6 wherein said release profile provides a blood serum concentration level of said doxycycline in said mammal of about 0.4 Rg/ml to about 0.8 Fg/ml.
8. A composition as described in clause 1 wherein said controlled-release agent is selected from the group consisting of an instantaneous release agent, a sustained-release agent, a delayed-release agent, and combinations thereof.
9. A composition according to clause 8 wherein said instantaneous release agent is a surfactant.
10. A composition according to clause 8 wherein said sustained release agent is selected from the group consisting of gels, waxes, fats, emulsifiers, polymers, starch, cellulose polymers, and combinations thereof.
11. A composition according to clause 10 wherein said cellulose polymers are selected from the group consisting of hydroxypropyl methyl cellulose (HPMC), hydroxyethyl cellulose (HEC), hydroxypropyl cellulose (HPC), carboxy methyl cellulose (CMC), and mixtures thereof.
12. A composition according to clause 8 wherein said delayed release agent is selected from the group consisting of a polymeric or biodegradable coating or matrix, or combinations thereof.
13. A composition as described in clause 1 wherein said association between said tetracycline compound and said controlled-release agent is selected from the group consisting of physical association, chemical association and combinations thereof.
14. A method of treating a mammal with a tetracycline compound comprising administering to said mammal an antibiotic tetracycline compound associated with a controlled-release matrix having at least one controlled-release agent to provide a release profile having a nonantibiotic activity over a pre-selected time period.
15. A method as described in clause 14 wherein said release profile provides a blood serum concentration level of said tetracycline compound in said mammal of about 0.1 to about 1.0 llg/ml.
16. A method as described in clause 15 wherein said release profile provides a blood serum concentration level of said tetracycline compound in said mammal of about 0.3 to about 0.8 llg/ml.
17. A method as described in clause 14 wherein said release profile is maintained at a substantially constant rate for between about 6-24 hours.
18. A method as described in clause 14 wherein said antibiotic tetracycline compound is selected from the group consisting of tetracycline, doxycycline, demeclocycline, minocycline, and lymecycline.
19. A method as described in clause 18 wherein said tetracycline compound is doxycycline.
20. A method as described in clause 19 wherein said release profile provides a blood serum concentration level of said doxycycline in said mammal of about 0.4 ug/ml to about 0.8 llg/ml.
21. A method as described in clause 14 wherein said control release agent is selected from the group consisting of an instantaneous release agent, a sustainedrelease agent, a delayed-release agent, and combinations thereof.
22. A method according to clause 21 wherein said instantaneous release agent is a surfactant.
23. A method according to clause 21 wherein said sustained release agent is selected from the group consisting of gels, waxes, fats, emulsifiers, polymers, starch, cellulose polymers, and combinations thereof.
24. A method according to clause 23 wherein said cellulose polymers are selected from the group consisting of hydroxypropyl methyl cellulose (HPMC), hydroxyethyl cellulose (HEC), hydroxypropyl cellulose (HPC), carboxy methyl cellulose (CMC), and mixtures thereof.
25. A method according to clause 21 wherein said delayed release agent is selected from the group consisting of a polymeric or biodegradable coating or matrix, or combinations thereof.
26. A method as described in clause 14 wherein said association between said tetracycline compound and said controlled-release agent is selected from the group consisting of physical association, chemical association and combinations thereof.
27. A method according to clause 14 wherein said tetracycline compound associated with a controlled-release matrix having at least one controlled-release agent are formed into a tablet.
28. A method according to clause 14 wherein said administration to said mammal is enteral administration.
29. A unit dosage for controlled delivery of a tetracycline comprising: a. an antibiotic tetracycline compound, and b. at least one controlled-release agent; and said tetracycline compound associated with said at least one controlled-release agent to provide a tetracycline-release-profile in said mammal, whereby said mammal is treated with said tetracycline substantially without antibiotic activity.
30. A unit dosage as described in clause 29 which is a capsule.
31. A unit dosage as described in clause 29 which is a tablet.
32. A unit dosage as described in clause 29 wherein said release profile provides a blood serum concentration level of said tetracycline compound in said mammal of about 0.1 llg/ml to about 1.0 llg/ml.
33. A unit dosage as described in clause 32 wherein said blood serum concentration level of said tetracycline compound in said mammal is between about 0.3 llg/ml to about 0.8 Fg/ml.
34. A unit dosage as described in clause 29 wherein said release profile is maintained at a substantially constant rate for between about 6-24 hours.
35. A unit dosage as described in clause 29 wherein said antibiotic tetracycline compound is selected from the group consisting of tetracycline, doxycycline, demeclocycline, minocycline, and lymecycline.
36. A unit dosage as described in clause 35 wherein said tetracycline compound is doxycycline.
37. A unit dosage as described in clause 36 wherein said release profile provides a blood serum concentration level of said tetracycline compound in said mammal of about 0.4 llg/ml to about 0.8 llg/ml.

## Claims

1. A composition for delivering a tetracycline compound to a mammal comprising:
a. an antibiotic tetracycline compound, and
b. at least one controlled-release agent;
wherein the composition provides a tetracycline-release-profile in a mammal.

2. A composition according to claim 1 wherein said release profile provides a blood serum concentration level of said tetracycline compound in said mammal of 0.1 µg/ml to 1.0 µg/ml, and preferably of 0.3 µg/ml to 0.8 µg/ml.

3. A composition according to claim 1 or 2, wherein said release-profile is maintained at a substantially constant rate for between 6-24 hours.

4. A composition according to any of the preceding claims wherein said antibiotic tetracycline compound is selected from the group consisting of tetracycline, doxycycline, demeclocycline, minocycline, and lymecycline.

5. A composition according to claim 4, wherein said tetracycline compound is doxycycline.

6. A composition according to claim 5, wherein said release profile provides a blood serum concentration level of said tetracycline compound in said mammal of 0.4 µg/ml to 0.8 µg/ml.

7. A composition according to any of the preceding claims, wherein said controlled-release agent is selected from the group consisting of an instantaneous release agent, a sustained-release agent, a delayed-release agent, and combinations thereof.

8. A composition according to claim 7, wherein said instantaneous release agent is a surfactant.

9. A composition according to claim 7, wherein said sustained release agent is selected from the group consisting of gels, waxes, fats, emulsifiers, polymers, starch, cellulose polymers, and combinations thereof.

10. A composition according to claim 9, wherein said cellulose polymers are selected from the group consisting of hydroxypropyl methyl cellulose (HPMC), hydroxyethyl cellulose (HEC), hydropropyl cellulose (HPC), carboxy methyl cellulose (CMC), and mixtures thereof.

11. A composition according to claim 7, wherein said delayed release agent is selected from the group consisting of a polymeric or biodegradable coating or matrix, or combinations thereof.

12. A composition according to any of the preceding claims wherein said association between said tetracycline compound and said controlled-release agent is selected from the group consisting of physical association, chemical association and combinations thereof.

13. A composition according to any of the preceding claims for treatment of a mammal with a tetracycline compound to provide a release profile having a nonantibiotic activity over a pre-selected time period.

14. A composition according to claim 13 wherein said tetracycline compound associated with a controlled-release matrix having at least one controlled-release agent are formed into a tablet.

15. A composition according to claim 14 for enteral administration.

16. A unit dosage for controlled delivery of a tetracycline comprising a composition according to any of the preceding claims.

17. A unit dosage according to claim 16 which is a capsule or a tablet.
